(19) Europäisches Patentamt

European Patent Office

Office européen des brevets

(11) **EP 0 992 284 B1**

(12) **EUROPÄISCHE PATENTSCHRIFT**

(45) Veröffentlichungstag und Bekanntmachung des Hinweises auf die Patenterteilung:
**22.02.2006 Patentblatt 2006/08**

(51) Int Cl.:
*B01J 21/04* (2006.01)　　*B01J 23/40* (2006.01)
*C10G 45/40* (2006.01)　　*C07C 5/02* (2006.01)

(21) Anmeldenummer: **99116473.2**

(22) Anmeldetag: **21.08.1999**

(54) **Katalysator und Verfahren zur Selektivhydrierung ungesättigter Verbindungen in Kohlenwasserstoffströmen**

Catalyst and process for the selective hydrogenation of unsaturated components in hydrocarbon streams

Catalyseur et procédé pour l'hydrogénation séléctive des composés insaturés aux courants d'hydrocarbures

(84) Benannte Vertragsstaaten:
**AT BE CH CY DE DK ES FI FR GB GR IE IT LI LU MC NL PT SE**

(30) Priorität: **29.08.1998 DE 19839459**

(43) Veröffentlichungstag der Anmeldung:
**12.04.2000 Patentblatt 2000/15**

(73) Patentinhaber: **BASF Aktiengesellschaft 67056 Ludwigshafen (DE)**

(72) Erfinder:
• **Meyer, Gerald, Dr.**
  **67067 Ludwigshafen (DE)**
• **Schwab, Ekkehard, Dr.**
  **67434 Neustadt (DE)**
• **Hesse, Michael, Dr.**
  **67549 Worms (DE)**
• **Trübenbach, Peter, Dr.**
  **67065 Ludwigshafen (DE)**
• **Müller, Hans-Joachim, Dr.**
  **9E The Manhattan,**
  **Hongkong (CN)**

(56) Entgegenhaltungen:
**FR-A- 2 584 734**　　　　**US-A- 3 635 841**

• **CHANG JUNE-CHENG; CHOU TSE-CHUAN: "Selective hydrogenation of isoprene over alumina-supported Pd catalysts: Effects of support on the catalytic performance" JOURNAL OF THE CHINESE INSTITUTE OF CHEMICAL ENGINEERS, Bd. 28, Nr. 5, September 1997 (1997-09), Seiten 329-339, XP000869962 Hsinchu, Taiwan**
• **DATABASE WPI Section Ch, Week 199408 Derwent Publications Ltd., London, GB; Class H04, AN 1994-057937 XP002127260 & CN 1 071 443 A (CHEM INST LANZHOU CHEM IND CORP), 28. April 1993 (1993-04-28)**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft edelmetallhaltige Katalysatoren auf einem Aluminiumoxidträger und Verfahren zur Selektivhydrierung ungesättigter Verbindungen in diese enthaltenden Kohlenwasserstoffströmen unter Verwendung dieser Katalysatoren.

In Raffinerien und petrochemischen Anlagen werden in großem Umfang Kohlenwasserstoffströme erzeugt, gelagert und verarbeitet. In diesen Kohlenwasserstoffströmen sind häufig ungesättigte Verbindungen vorhanden, deren Anwesenheit insbesondere bei Verarbeitung und/oder Lagerung bekanntermaßen zu Problemen führt, oder die nicht das gewünschte Wertprodukt darstellen, und die daher unerwünschte Komponenten der entsprechenden Kohlenwasserstoffströme sind. Allgemeine Übersichten über derartige Probleme bei Steamcrackern und übliche Lösungen gaben beispielsweise H.-M. Allmann, Ch. Herion und P. Polanek in ihrem vortrag "Selective Hydrogenations and Purifications in the Steamcracker Downstream Treatment" auf der DGMK-Konferenz "Selective Hydrogenation and Dehydrogenation" am 11. und 12. November 1993 in Kassel, Deutschland, dessen Manuskript auch im Tagungsbericht 9305 der DGMK Deutsche Wissenschaftliche Gesellschaft für Erdöl, Erdgas und Kohle e. V., Hamburg, S. 1 - 30, erschienen ist (ISSN 0938-068X, ISBN 3-928164-61-9), und M. L. Derrien in: L. Cerveny (Hrsg.), Stud. Surf. Sci. Catal., Bd. 27, S. 613 - 666, Elsevier, Amsterdam 1986.

Üblicherweise ist in C2-Strömen von Steamcrackern die Nebenkomponente Acetylen unerwünscht, in C3-Strömen sind die Nebenkomponenten Propin und Allen unerwünscht und in C4-Strömen sind die Nebenkomponenten 1- und 2-Butin, 1,2-Butadien und Vinylacetylen unerwünscht, wenn 1,3-Butadien als Wertprodukt gewonnen und weiterverarbeitet werden soll, sowie die genannten Nebenkomponenten und 1,3-Butadien selbst in den Fällen, in denen 1-Buten, 2-Buten (in der cis- und/oder der trans-Form) oder Isobuten die gewünschten Produkte sind. Bei der Verarbeitung von C5+-Strömen ("C5+":Kohlenwasserstoffe mit mindestens 5 C-Atomen, "Pyrolysebenzin") sind Di- und Polyene wie Pentadien und Cyclopentadien, Alkine und/oder Aromaten mit ungesättigten Substituenten wie Phenylacetylen und Styrol bei der Gewinnung und Verarbeitung von Aromaten oder Vergaserkraftstoff unerwünscht.

Bei Kohlenwasserstoffströmen, die einem FCC-Cracker oder einem Reformer statt einem Steamcracker entstammen, treten analoge Probleme auf. Eine allgemeine Übersicht über solche Probleme, speziell bei C4- und C5+-Strömen aus FCC-Crackern gaben beispielsweise J. P. Boitiaux, C. J. Cameron, J. Cosyns, F. Eschard und P. Sarrazin in ihrem Vortrag "Selective Hydrogenation Catalysts and Processes: Bench to Industrial Scale" auf der DGMK-Konferenz "Selective Hydrogenation and Dehydrogenation" am 11. und 12. November 1993 in Kassel, Deutschland, dessen Manuskript auch im Tagungsbericht 9305 der DGMK Deutsche Wissenschaftliche Gesellschaft für Erdöl, Erdgas und Kohle e. V., Hamburg, S. 49 - 57, erschienen ist (ISSN 0938-068X, ISBN 3-928164-61-9).

Im Allgemeinen sind daher aus Kohlenwasserstoffströmen zumeist ungesättigte Verbindungen mit Dreifachbindungen (Alkine) und/oder zweifach ungesättigte Verbindungen (Diene) und/oder andere zwei- oder mehrfach ungesättigte Verbindungen (Polyene, Allene, Alkinene) und/oder aromatische Verbindungen mit einem oder mehreren ungesättigten Substituenten (Phenylalkene und Phenylalkine) zu entfernen, um die gewünschten Produkte wie Ethylen, Propylen, 1-Buten, Isobuten, 1,3-Butadien, Aromaten oder Vergaserkraftstoff in der geforderten Qualität zu erhalten. Nicht jede ungesättigte Verbindung ist jedoch immer eine unerwünschte Komponente, die aus dem fraglichen Kohlenwasserstoffstrom zu entfernen ist. Beispielsweise ist 1,3-Butadien, wie oben bereits angedeutet, je nach Anwendungsfall eine unerwünschte Nebenkomponente oder das gewünschte Wertprodukt.

Die Entfernung unerwünschter ungesättigter Verbindungen aus diese enthaltenden Kohlenwasserstoffströmen geschieht häufig durch Selektivhydrierung mancher oder aller der unerwünschten ungesättigten Verbindungen im entsprechenden Kohlenwasserstoffstrom, vorzugsweise durch Selektivhydrierung zu nicht störenden, höher gesättigten Verbindungen und in besonders bevorzugter Weise zu den Wertprodukte darstellenden Komponenten des Kohlenwasserstoffstroms. Beispielsweise wird in C2-Strömen Acetylen zu Ethylen hydriert, in C3-Strömen Propin und Allen zu Propylen, in C4-Strömen Butin zu Butenen, Vinylacetylen zu 1,3-Butadien und/oder 1,3-Butadien zu Butenen und in C5+-Strömen Phenylacetylen und Styrol zu Ethylbenzol, Cyclopentadien zu Cyclopenten und Pentadien zu Penten.

Typischerweise sind solche Verbindungen bis auf Restgehalte von wenigen Gew.-ppm zu entfernen. Die ("Über-")Hydrierung zu Verbindungen, die höher gesättigt ist als das gewünschte Wertprodukt und/oder die parallele Hydrierung eines eine oder mehrere Mehrfachbindungen enthaltenden Wertprodukts zur entsprechenden höher oder vollständig gesättigten Verbindung sollen aufgrund des damit verbundenen Wertverlusts jedoch möglichst vermieden werden. Die Selektivität der Hydrierung der unerwünschten ungesättigten Verbindungen muß daher möglichst hoch sein. Zusätzlich sind im Allgemeinen eine ausreichend hohe Aktivität des Katalysators und eine lange Standzeit erwünscht. Gleichzeitig soll der Katalysator möglichst auch keine anderen unerwünschten Nebenreaktionen bewirken, beispielsweise ist eine Katalyse der Isomerisierung von 1-Buten zu 2-Buten mit Ausnahme spezieller Sonderfälle möglichst zu vermeiden. Üblicherweise werden Edelmetall-Trägerkatalysatoren eingesetzt, in denen Edelmetall auf einem Katalysatorträger abgeschieden ist. Häufig wird Palladium als Edelmetall verwendet, der Träger ist im Allgemeinen ein poröses anorganisches Oxid, beispielsweise Kieselerde, Alumosilikat, Titandioxid, Zirkoniumdioxid, Zinkaluminat, Zinktitanat und/oder Mischungen solcher Träger, meist werden jedoch Aluminiumoxid oder Siliciumdioxid verwendet. Weiterhin können Promotoren oder andere Zusatzstoffe enthalten sein. Verfahren zur selektiven Hydrierung ungesättigter Verbindungen in diese enthaltenden Kohlenwasserstoffströmen sind sowohl als Flüssigphasenhydrierung oder gemischte Gas/Flüssigphasenhydrierung, in Riesel- oder Sumpffahrweise, wie auch als reine Gasphasenhydrierung bekannt, wobei verschiedene verfahrenstechnische Maßnahmen zur Verbesserung der Selektivität publiziert wurden.

Beispielsweise lehrt EP-A 87980 ein derartiges Verfahren in einem Festbettreaktor, bei dem der Hydrierwasserstoff an mindestens zwei Stellen entlang des Reaktors zugeführt wird, wodurch eine höhere Selektivität erreicht wird. EP-A 523482 offenbart die Durchführung eines solchen Verfahrens in zwei hintereinandergeschalteten Reaktionszonen, wodurch die unerwünschte Überhydrierung zu n-Butan weitgehend unterdrückt wird und die Gesamtselektivtität ebenfalls steigt. EP-A 81041 lehrt, dass der Zusatz von Kohlenmonoxid die Hydrier- und Isomerisierungaktivität des als Katalysatormetall verwendeten Palladiums herabsetzt und so die Selektivität erhöht. JP-A 01-110594 lehrt den Zusatz weiterer Elektronendonor-Verbindungen, entweder in Form einer Dotierung des Katalysators, etwa mit Alkalimetallen, oder in Form eines Zusatzes zum Reaktionsgemisch, etwa von Alkoholen, Ethern oder stickstoffhaltigen Verbindungen.

Auch die Verwendung von Promotoren oder Dotierstoffen zusätzlich zum eigentlich hydrieraktiven Katalysatormetall ist bekannt.

So lehren J. P. Boitiaux, J. Cosyns, M. Derrien und G. Léger in Hydrocarbon Processing, 1985 (3), S.51-59, die Verwendung bimetallischer Katalysatoren, insbesondere solcher, die Metalle der Gruppe VIII (aktuelle IUPAC-Nomenklatur: Gruppen 8, 9 und 10), speziell Palladium, und Metalle der Gruppe IB (aktuelle IUPAC-Nomenklatur: Gruppe 11) des Periodensystems der Elemente enthalten. EP-A 564328 und EP-A 564329 lehren die Verwendung von Katalysatoren, die Metalle der Gruppe VIII, speziell Palladium, und Metalle der Gruppe IIIA (aktuelle IUPAC-Nomenklatur: Gruppe 3), speziell Indium oder Gallium, enthalten, und ihre Verwendung. EP-A 89252 offenbart ein Verfahren zur Herstellung eines Palladium und Gold enthaltenden Trägerkatalysators und dessen Anwendung. US-A 5,475,173 offenbart einen Palladium, Silber und Alkalifluorid enthaltenden Katalysator. EP-A 722776 offenbart einen besonders gegen Verunreinigungen durch Schwefel resistenten Katalysator, der aus Palladium, mindestens einem Alkalifluorid und optional Silber auf einem anorganischen Träger wie $TiO_2$, $ZrO_2$ oder vorzugsweise $Al_2O_3$ besteht. EP-A 211381 lehrt die Verwendung eines Katalysators, der ein Meta11 der Gruppe VIII des Periodensystems der Elemente, vorzugsweise Pt, mindestens ein unter Blei, Zinn und Zink gewähltes Metall und einen anorganischen Träger umfaßt. Der dort bevorzugte Katalysator ist Platin auf einem Zinkspinell-Träger ($ZnAl_2O_4$). US-A 4,260,840 lehrt Palladium und Chrom enthaltenden Katalysatoren, die sich durch besonders geringe Isomerisierungsneigung auszeichnen.

Es ist auch möglich, die Eigenschaften des verwendeten Katalysators nicht nur durch verfahrenstechnische Maßnahmen oder die Verwendung bestimmter Zusatzstoffe zu beeinflussen, sondern auch durch die Art des Trägers und die Art der Verteilung der Aktivmasse auf der inneren und äußeren Oberfläche des Trägers.

So offenbart DE-A 31 19 850 die Verwendung eines Katalysators, der aus Palladium und Silber auf einem Träger aus $SiO_2$ mit einer BET-Oberfläche im Bereich von 10 bis 200 $m^2$/g oder auf einem Träger aus $Al_2O_3$ mit einer BET-Oberfläche von weniger als 100 $m^2$/g besteht. DE-A 20 59 978 lehrt Palladiumkatalysatoren auf einem Tonerdeträger ("Tonerde" ist ein gebräuchliches Synonym für Aluminiumoxid). Der Träger weist eine BET-Oberfläche von rund 120 $m^2$/g auf und wird vor Abscheidung des Palladiums zunächst einer Wasserdampf-Behandlung bei 110-300°C unterzogen und anschließend bei 500-1200°C kalziniert.

K. H. Walter, W. Droste, D. Maschmeyer und F. Nierlich wiesen in ihrem Vortrag: "The Hüls Process for Selective Hydrogenation of Butadiene in crude C4s - Development and Technical Application" auf der DGMK-Konferenz "Selective Hydrogenation and Dehydrogenation" am 11. und 12. November 1993 in Kassel, Deutschland, dessen Manuskript auch im obengenannten Tagungsbericht S. 31 - 48, erschienen ist, auf die Bedeutung der Abstimmung zwischen Diffusions- und Reaktionsgeschwindigkeit im Katalysatorkorn für das Verfahren hin,und lehrten Katalysatoren, in denen Palladium praktisch ausschließlich auf der äußeren Oberfläche der Trägerpartikel konzentriert ist ("Schalenkatalysatoren"). EP-A 780155 offenbart einen Katalysator, der aus Palladium und einem Metall der Gruppe IB des Periodensystems der Elemente auf einem $Al_2O_3$-Träger besteht, wobei mindestens 80 % des Pd und 80 % des Metalls der Gruppe IB in den Volumenteilen des Katalysatorkorns konzentriert sind, die vom Radius des Katalysatorkorns und einer Strecke vom Mittelpunkt, die 0,8 mal diesem Radius entspricht, begrenzt werden. EP-A 653243 lehrt Katalysatoren, in sich die Aktivkomponenten überwiegend in den Meso- und Makroporen des Trägers befinden.

EP-A 576828 lehrt Katalysatoren zur selektiven Hydrierung ungesättigter Verbindungen in Kohlenwasserstoffströmen, die aus Edelmetall oder Edelmetallverbindungen auf einem speziellen $Al_2O_3$-Träger bestehen, wobei der Katalysator durch ein bestimmtes Röntgenbeugungsmuster definiert ist. Dieses Röntgenbeugungsmuster wird überwiegend durch den Träger bestimmt und ist in diesem Fall typisch für die $\eta$-$Al_2O_3$ und/oder $\gamma$-$Al_2O_3$-Modifikationen. US 3,615,207 und US 3,635,841 lehren Palladiumkatalysatoren auf Trägern aus $\delta$-und $\theta$-Aluminiumoxiden, die frei von $\alpha$- und $\gamma$-Aluminiumoxiden sind, und ihre Verwendung zur Hydrierung von Alkylanthrachinonen.

J.-C. Chang und T.C. Chou beschreiben in J. Chin. Inst. Chem. Engrs., Vol. 28, No. 5, (1997), Seiten 329 ff. Pd-haltige Katalysatoren auf $Al_2O_3$-Trägern und ihre Verwendung zur Isoprenhydrierung. CN 1071443 A offenbart einen Katalysator, der $\alpha$- und $\delta$-$Al_2O_3$ und Pd umfasst.

Die Anforderungen an Katalysatoren und Verfahren zur selektiven Hydrierung unerwünschter ungesättigter Verbindungen in diese enthaltenden Kohlenwasserstoffströmen im Hinblick auf die Verringerung des Restgehalts an unerwünschten ungesättigten Verbindungen nach der Hydrierung und auf die Erhöhung der Selektivität steigen ständig. Die bekannten Verfahren und Katalysatoren arbeiten zwar schon auf sehr hohem technischem Stand, sind aber angesichts der steigenden Anforderungen immer noch unbefriedigend. Es bestand daher die Aufgabe, einen verbesserten Katalysator und ein verbessertes Verfahren zur selektiven Hydrierung ungesättigter Verbindungen in diese enthaltenden Kohlenwasserstoffströmen zu finden.

Dementsprechend wurde ein Katalysator gefunden, der mindestens ein hydrieraktives Metall auf einem Aluminiumoxidträger umfaßt und ungebraucht im Röntgendiffraktogramm Reflexe zeigt, die den folgenden Netzebenenabständen entsprechen:

| Netzebenenabstand d [$10^{-10}$ m] | relative Intensität $I/I_o$ |
| --- | --- |
| 4,52 | 0,05 bis 0,1 |
| 2,85 | 0,35 bis 0,45 |
| 2,73 | 0,65 bis 0,8 |
| 2,44 | 0,45 bis 0,55 |
| 2,31 | 0,35 bis 0,45 |
| 2,26 | 0,35 bis 0,45 |
| 2,02 | 0,45 bis 0,6 |
| 1,91 | 0,3 bis 0,4 |
| 1,80 | 0,1 bis 0,25 |
| 1,54 | 0,25 bis 0,35 |
| 1,51 | 0 bis 0,35 |
| 1,49 | 0,2 bis 0,3 |
| 1,45 | 0,25 bis 0,35 |
| 1,39 | 1 |

Weiterhin wurden ein Verfahren zur Herstellung dieses Katalysators sowie Verfahren zur selektiven Hydrierung ungesättigter Verbindungen in diese enthaltenden Kohlenwasserstoffströmen unter Verwendung des erfindungsgemäßen Katalysators gefunden.

Röntgenbeugungsdiagramme sind charakteristisch für die spezifische Struktur des untersuchten Materials. Die Struktur des erfindungsgemäßen Katalysators ist durch das Auftreten der oben angegebene Reflexe hinreichend definiert und von der bekannter Katalysatoren verschieden. Zusätzlich zu den oben angegebenen kennzeichnenden Reflexen können im Röntgenbeugungsdiagramm ein oder mehrere Reflexe in beliebiger Intensität für die Netzebenenabstände (alle in der Einheit [$10^{-10}$ m]) 3,48, 2,55, 2,38, 2,09, 1,78, 1,74, 1,62, 1,60, 1,57, 1,42, 1,40 und/oder 1,37 auftreten. Weiterhin können im Röntgenbeugungsdiagramm des erfindungsgemäßen Katalysators noch beliebige weitere Reflexe auftreten.

Der erfindungsgemäße Katalysator weist besonders in der selektiven Hydrierung von Alkinenen zu Alkadienen, bei der selektiven Hydrierung von Alkinen, Alkinenen und Alkadienen zu Alkenen und/oder bei der selektiven Hydrierung von Phenylalkinen zu Phenylalkenen und/oder Phenylalkanen und/oder zur selektiven Hydrierung von Phenylalkenen zu Phenylalkanen hervorragende Eigenschaften auf, insbesondere eine hohe Selektivität sowohl bei Durchführung des Verfahrens mit den Einsatzstoffen in der Flüssigphase oder in der gemischten Flüssig- und Gasphase als auch in der Gasphase. Bei Verwendung des erfindungsgemäßen Katalysators treten die unerwünschte Überhydrierung zu den gesättigten Kohlenwasserstoffen, beispielsweise Propan, n-Butan oder den C5+-Alkanen, und die bei der Selektivhydrierung von C4-Strömen unerwünschte Isomerisierung von 1-Buten zu 2-Buten nur in überraschend geringem Umfang auf. Zusätzlich ist der Katalysator vergleichsweise aktiv und kann über vergleichsweise lange Zeitdauern betrieben werden. Der erfindungsgemäße Katalysator zeigt diese vorteilhaften Eigenschaften auch ohne weitere verfahrenstechnische Maßnahmen, beispielsweise ohne Zusatz von Kohlenmonoxid oder Alkoholen, Ethern oder stickstoffhaltigen Verbindungen.

Der Träger besteht im wesentlichen aus Aluminiumoxid, das neben unvermeidbaren Verunreinigungen in gewissem Umfang auch andere Zusätze enthalten kann, solange sich die Struktur des Katalysators, die durch das oben angegebene Röntgenbeugungsdiagramm charakterisiert ist, dadurch nicht ändert. Beispielsweise können andere anorganische Oxide wie oxide von Metallen der 2., 3., 4., 13. und 14. Gruppe des Periodensystems der Elemente enthalten sein, insbesondere Siliciumdioxid, Titandioxid, Zirkondioxid, Zinkoxid, Magnesiumoxid und Calciumoxid. Der maximale Gehalt des Trägers an solchen, von Aluminiumoxid verschiedenen Oxiden ist vom tatsächlich vorhandenen Oxid abhängig, aber im Einzelfall leicht anhand des Röntgenbeugungsdiagramms zu ermitteln, da eine Änderung der Struktur mit einer signifikanten Änderung des Röntgenbeugungsdiagramms einhergeht. Im Allgemeinen liegt der Gehalt an solchen, von Aluminiumoxid verschiedenen Oxiden unterhalb von 50 Gew.-%, vorzugsweise unterhalb von 30 Gew.-% und in besonders bevorzugter Weise unterhalb von 10 Gew.-%.

Zur Herstellung des Trägers wird ein geeigneter aluminiumhaltiger Rohstoff, vorzugsweise Böhmit, mit einem Peptisierungsmittel wie Wasser, verdünnter Säure oder verdünnter Base peptisiert. Als Säure wird beispielsweise eine Mineralsäure wie etwa Salpetersäure oder eine organische Säure wie etwa Ameisensäure verwendet, als Base wird eine anorganische Base wie etwa Ammoniak verwendet. Die Säure oder Base wird im Allgemeinen in Wasser gelöst. Vorzugsweise werden als Peptisierungsmittel Wasser oder verdünnte wäßrige Salpetersäure verwendet. Die Konzentration des nichtwäßrigen Anteils im Peptisierungsmittel beträgt im Allgemeinen 0 bis 10 Gew.-%, vorzugsweise 0 bis 7 Gew.-% und in besonders bevorzugter Weise 0 bis 5 Gew.-%. Anschließend an die Peptisierung wird der Träger verformt, danach werden die Formlinge getrocknet und kalziniert.

Böhmit ($\gamma$-AlO(OH)) ist ein verbreitetes Handelsprodukt, kann aber auch in bekannter Weise unmittelbar vor der eigentlichen Trägerherstellung durch Fällung aus einer Lösung eines Aluminiumsalzes, beispielsweise Aluminiumnitrat, mit Base, Abtrennen, Waschen, trocknen und Kalzinieren des gefällten Feststoffs hergestellt werden. Vorteilhafterweise wird Böhmit in der Form eines Pulvers verwendet. Ein geeignetes handelsübliches Böhmit-Pulver ist beispielsweise Versal® 250, das von Euro Support, Amsterdam, erhältlich ist. Der Böhmit wird mit dem Peptisierungsmittel behandelt, indem er mit dem Peptisierungsmittel angefeuchtet und intensiv durchmischt wird, beispielsweise in einem Kneter, Mischer oder Kollergang. Die Peptisierung wird fortgesetzt, bis die Masse gut verformbar ist. Anschließend wird die Masse mittels üblicher Methoden zu den gewünschten Trägerformkörpern verformt, beispielsweise durch Strangpressen, Extrudieren, Tablettieren oder Agglomerieren. Zur Verformung ist jede bekannte Methode geeignet, falls nötig, können übliche Zusätze verwendet werden. Beispiele für solche Zusätze sind Extrudier- oder Tablettierhilfsmittel wie Polyglykole oder Graphit.

Es ist ferner möglich, der Trägerrohmasse vor der Verformung Zusätze beizumischen, die in bekannter Weise als Ausbrennstoffe die Porenstruktur des Trägers nach der Kalzination beeinflussen, beispielsweise Polymere, Faserstoffe, natürliche Ausbrennstoffe wie Nußschalenmehle oder andere übliche Zusätze. Bevorzugt ist die Verwendung von Böhmit in einer Korngrößenverteilung und die Zugabe von Ausbrennstoffen, die zu einer Porenradienverteilung des fertigen Trägers führt, bei der 50 - 90 Vol.-% des gesamten Porenvolumens in Form von Poren mit einem mittleren Durchmesser im Bereich von 0,01 bis 0,1 Mikrometer und 10 bis 50 Vol.-% des gesamten Porenvolumens in Form von von Poren mit einem mittleren Durchmesser im Bereich von 0,1 bis 1 Mikrometer vorliegen. Die hierzu notwendigen Maßnahmen sind dem Fachmann bekannt.

Im Anschluß an die Verformung werden die Formkörper in üblicher Weise getrocknet, im Allgemeinen bei einer Temperatur oberhalb von 60 °C, vorzugsweise oberhalb von 80 °C und in besonders bevorzugter Weise oberhalb von 100 °C, beispielsweise bei einer Temperatur im Bereich von 120 °C bis 300 °C. Die Trocknung wird fortgesetzt, bis in Formkörpern vorhandenes Wasser im wesentlichen vollständig aus den Formkörpern entwichen ist, was im Allgemeinen nach einigen Stunden der Fall ist. Übliche Trocknungsdauern liegen im Bereich von einer bis 30 Stunden und sind von der eingestellten Trocknungstemperatur abhängig, höhere Temperatur verkürzt die Trocknungszeit. Die Trocknung kann durch Anwenden eines Unterdrucks weiter beschleunigt werden.

Im Anschluß an die Trocknung werden die Formkörper durch Kalzination zum fertigen Träger umgewandelt. Die Kalzinationstemperatur liegt im Bereich von 900 °C bis 1100 °C, vorzugsweise im Bereich von 950 °C bis 1050 °C und in besonders bevorzugter Weise im Bereich von 980 °C bis 1030 °C. Die Kalzinationsdauer liegt im Allgemeinen zwischen 0,5 und 5 Stunden, in bevorzugter Weise zwischen einer und 4 Stunden und in besonders bevorzugter Weise zwischen 1,5 und 3 Stunden. Die Kalzination erfolgt in einem üblichen Ofen, beispielsweise in einem Drehrohrofen, in einem Bandkalzinierer oder in einem Kammerofen. Die Kalzination kann sich ohne zwischenzeitliche Abkühlung der Formkörper direkt an die Trocknung anschließen. Die BET-Oberfläche des so hergestellten Trägers liegt üblicherweise im Bereich von 30-120 m$^2$/g. Die Oberfläche kann durch bekannte Methoden (insbesondere Verwendung feinteiligerer oder gröberer Ausgangsstoffe, Kalzinationsdauer und -temperatur) variiert werden. Bevorzugterweise liegt die BET-Oberfläche im Bereich 40 bis 100 m$^2$/g und in besonders bevorzugter Weise im Bereich von 60-90 m$^2$/g. wie die BET-Oberfläche kann auch das Porenvolumen auf bekannte Weise variiert werden, im Allgemeinen liegt es, mittels Quecksilberporosimetrie gemessen in einem Bereich von 0,3 bis 1,0 ml/g. Bevorzugterweise liegt es im Bereich von 0,4 bis 0,9 ml/g und in besonders bevorzugter Weise im Bereich von 0,5 bis 0,8 ml/g.

Nach der Kalzination werden auf dem so hergestellten Träger die Aktivmasse und gegebenenfalls weitere Zusatzstoffe abgeschieden.

Der Katalysator kann ein oder mehrere hydrieraktive Metalle, Zusatzstoffe und/oder Promotoren enthalten.

Als hydrieraktive Metalle sind im erfindungsgemäßen Katalysator vor allem die Metalle der 8., 9. und 10.

Gruppen des Periodensystems der Elemente geeignet, insbesondere Ruthenium, Rhodium, Palladium und/oder Platin. Besonders geeignet sind Platin und/oder Palladium, ganz besonders bevorzugt ist Palladium. Der Katalysator kann weiterhin alle für Katalysatoren zur selektiven Hydrierung mehrfach ungesättigter Verbindungen bekannten Zusatzstoffe und Promotoren enthalten. Beispielsweise können die erfindungsgemäßen Katalysatoren neben dem Metall oder den Metallen aus den 8., 9. und 10. Gruppen des Periodensystems der Elemente noch mindestens ein Metall aus der 11. Gruppe des Periodensystems der Elemente enthalten. In diesem Fall sind unter als Elemente der 11. Gruppe Kupfer und Silber bevorzugt, besonders bevorzugt ist Silber. Weiterhin enthält in diesem Fall der Katalysator in ganz besonders bevorzugter Weise Palladium und Silber.

Die Metalle können in reiner metallischer Form vorliegen, aber auch in Form von Verbindungen, beispielsweise in Form von Metalloxiden. Unter den Betriebsbedingungen eines Hydrierverfahrens liegen sie im Allgemeinen in Form von Metallen vor. Die Umwandlung etwaiger Oxide in Metalle kann in bekannter Weise vor dem Einsatz des Katalysators in einem Hydrierverfahren durch Vorreduzierung und, falls für Manipulationen mit dem vorreduzierten Katalysator nötig, anschließende oberflächliche Passivierung erfolgen.

Der Gehalt des Katalysators an Metall oder Metallen der 8., 9. und 10. Gruppen des Periodensystems der Elemente, insbesondere Palladium, beträgt im Allgemeinen mindestens 0,05 Gew.-%, bezogen auf die Gesamtmasse des Katalysators, bevorzugterweise mindestens 0,08 Gew.-% und in besonders bevorzugter Weise mindestens 0,1 Gew.-%. Im Allgemeinen liegt dieser Gehalt bei höchstens 2 Gew.-%, bevorzugterweise bei höchstens 1 Gew.-% und in besonders bevorzugter Weise bei höchstens 0,5 Gew.-%. Niedrigere oder höhere Gehalte sind zwar möglich, aber im Normalfall wegen zu niedriger Aktivität oder zu hohen Rohstoffkosten wirtschaftlich unbefriedigend.

Beispielsweise kann der erfindungsgemäße Katalysator 0,3 Gew.-% Palladium enthalten.

Der Gehalt des Katalysators an Metall oder Metallen anderer Gruppen als den 8., 9. und 10. Gruppen des Periodensystems der Elemente, insbesondere Metallen der 11. Gruppe, und ganz besonders von Silber und/oder Kupfer, beträgt - sofern solche Metalle vorhanden sind - im Allgemeinen mindestens 0,01 Gew.-%, bezogen auf die Gesamtmasse des Katalysators, bevorzugterweise mindestens 0,03 Gew.-% und in besonders bevorzugter weise mindestens 0,05 Gew.-%. Im Allgemeinen liegt dieser Gehalt bei höchstens 1 Gew.-%, bevorzugterweise bei höchstens 0,7 Gew.-% und in besonders bevorzugter Weise bei höchstens 0,5 Gew.-%. Niedrigere oder höhere Gehalte sind zwar möglich, aber im Normalfall wegen zu geringer Wirkung oder zu hohen Stoffkosten wirtschaftlich unbefriedigend.

Das Verhältnis der Mengen von hydrieraktivem Metall aus der 8., 9. und 10. Gruppen des Periodensystems der Elemente und Zusatz- oder Dotierstoffen ist ein im Einzelfall zu optimierender Parameter. Enthält der Katalysator Palladium und Silber oder Kupfer, liegt das Massenverhältnis von Silber oder Kupfer zu Palladium im Allgemeinen im Bereich von 0,1 bis 5, bevorzugterweise im Bereich von 0,15 bis 2 und in besonders bevorzugter Weise im Bereich von 0,2 bis 1. Enthält der Katalysator Palladium und Silber und Kupfer, liegen im Allgemeinen sowohl Silber als auch Kupfer jeweils in einem Massenverhältnis zu Palladium im Bereich von 0,1 bis 5, bevorzugterweise im Bereich von 0,15 bis 2 und in besonders bevorzugter Weise im Bereich von 0,2 bis 1 vor.

Beispielsweise kann der erfindungsgemäße Katalysator 0,2 Gew.-% Palladium und 0,1 Gew.-% Silber enthalten.

Die auf dem Träger abzuscheidenden Metalle, Zusatz- und/oder Dotierstoffe können mit jedem bekannten Verfahren auf den Träger aufgebracht werden, beispielsweise durch Beschichtung aus der Gasphase (chemical oder physical vapour deposition), die bevorzugte Methode ist jedoch die Tränkung mit einer Lösung der abzuscheidenden Substanzen und/oder Verbindungen, die sich im Zuge der weiteren Katalysatorherstellung in die abzuscheidenden Substanzen umwandeln. Die einzelnen abzuscheidenden Substanzen können einzeln und/oder in Teilmengen in mehreren Verfahrensschritten oder gemeinsam und vollständig in einem Verfahrensschritt abgeschieden werden. Bevorzugt ist die gemeinsame Abscheidung in einer Tränkstufe. Im Anschluß an die Tränkung oder nach den einzelnen Tränkstufen wird der getränkte Träger getrocknet, und durch Kalzinierung sowie gegebenenfalls andere bekannte Nachbehandlungsmethoden (beispielsweise Aktivierung und anschließende oberflächliche Passivierung) zum einsatzbereiten Katalysator umgewandelt.

Tränkverfahren zur Abscheidung von Aktivkomponenten, Zusatzstoffen und/oder Dotierstoffen auf einem Träger sind bekannt. Im Allgemeinen wird der Träger mit einer Lösung von Salzen der abzuscheidenden Komponenten getränkt, wobei das Volumen der Lösung so bemessen wird, dass die Lösung praktisch vollständig vom Porenvolumen des Trägers aufgenommen wird ("incipient wetness"-Methode). Die Konzentration der Salze in der Lösung wird so bemessen, dass nach Tränkung und Umwandlung des getränkten Trägers zum fertigen Katalysator die abzuscheidenden Komponenten in der gewünschten Konzentration auf dem Katalysator vorliegen. Die Salze werden so gewählt, dass sie keine bei der Katalysatorherstellung oder dessen späterer Verwendung störenden Rückstände hinterlassen. Meist werden Nitrate oder Ammoniumsalze verwendet.

Der Katalysator kann gegebenenfalls auch in Form eines Schalenkatalysators hergestellt werden, Methoden hierzu sind bekannt. Vorzugsweise sind in einem solchen Fall die Aktivkomponenten, Zusatzstoffen und/oder Dotierstoffen überwiegend in einer von der äußeren Oberfläche des Katalysatorformkörpers begrenzten Schale von höchstens 2000 Mikrometer Dicke konzentriert, in besonders bevorzugter Weise beträgt die Dicke der Schale höchstens 1000 Mikrometer.

Die Herstellung des erfindungsgemäßen Katalysators erfolgt vorzugsweise unter einstufiger Tränkung des Trägers nach der incipient wetness-Methode mit einer salpetersauren Lösung der Nitrate der abzuscheidenden Metalle. Die Konzentration der verwendeten Salpetersäure ist mindestens so hoch, dass eine klare Lösung vorliegt. Im Allgemeinen liegt der pH-Wert der Lösung bei höchstens 5, bevorzugterweise bei höchstens 2 und in besonders bevorzugter Weise bei höchstens 1.

Nach der Tränkung wird der getränkte Träger in üblicher Weise getrocknet, im Allgemeinen bei einer Temperatur oberhalb von 60 °C, vorzugsweise oberhalb von 80 °C und in besonders bevorzugter Weise oberhalb von 100 °C, beispielsweise bei einer Temperatur im Bereich von 120 °C bis 300 °C. Die Trocknung wird fortgesetzt, bis im getränkten Träger vorhandenes Wasser im wesentlichen vollständig entwichen ist, was im Allgemeinen nach einigen Stunden der Fall ist. Übliche Trocknungsdauern liegen im Bereich von einer bis 30 Stunden und sind von der eingestellten Trocknungstemperatur abhängig, höhere Temperatur verkürzt die Trocknungszeit. Die Trocknung kann durch Anwenden eines Unterdrucks weiter beschleunigt werden.

Im Anschluß an die Trocknung wird in üblicher Weise durch Kalzinierung der Katalysator hergestellt. Diese Kalzinierung dient im wesentlichen der Umwandlung der aufgetränkten Salze in die abzuscheidenden Komponenten oder Vorläufer solcher Komponenten und unterscheidet sich insofern von der oben beschriebenen Kalzinierung, die der Herstellung des Trägermaterials und der Trägerstruktur dient. Im Falle der Auftränkung von Metallnitraten werden bei dieser Kalzinierung im wesentlichen die Nitrate in Metalle und/oder Metalloxide, die im Katalysator verbleiben, und nitrose Gase, die entweichen, zersetzt.

Die Kalzinationstemperatur liegt im Allgemeinen im Bereich von 250 °C bis 900 °C, vorzugsweise im Bereich von 280 °C bis 800 °C und in besonders bevorzugter Weise im Bereich von 300 °C bis 700 °C. Die Kalzinationsdauer liegt im Allgemeinen zwischen 0,5 und 20 Stunden, in bevorzugter Weise zwischen 0,5 und 10 Stunden und in besonders bevorzugter Weise zwischen 0,5 und 5 Stunden.
Die Kalzination erfolgt in einem üblichen Ofen, beispielsweise in einem Drehrohrofen, in einem Bandkalzinierer oder in einem Kammerofen. Die Kalzination kann sich ohne zwischenzeitliche Abkühlung des getränkten und getrockneten Trägers direkt an die Trocknung anschließen.

Nach der Kalzination ist der Katalysator prinzipiell einsatzbereit. Falls erforderlich oder gewünscht, wird er vor seinem Einsatz zur Selektivhydrierung in bekannter Weise durch vorreduzierung aktiviert und gegebenenfalls auch wieder oberflächlich passiviert.

Die erfindungsgemäßen Verfahren zur selektiven Hydrierung zeichnen sich durch den Einsatz des erfindungsgemäßen Katalysators aus. Die erfindungsgemäßen Hydrierverfahren unter Einsatz des erfindungsgemäßen Katalysators werden im Allgemeinen genauso wie die bekannten, denselben Zwecken dienenden, heterogen katalysierten Hydrierverfahren durchgeführt. Sie können als heterogen katalysierte Gasphasenverfahren, bei denen sich sowohl der Kohlenwasserstoffstrom wie auch der Hydrierwasserstoff in der Gasphase befinden, oder als heterogen katalysierte Gas/Flüssigphasenverfahren, bei denen der Kohlenwasserstoffstrom zumindest teilweise in flüssiger Phase und Wasserstoff in der Gasphase und/oder in gelöster Form in der Flüssigphase vorliegen, durchgeführt werden. Die einzustellenden Parameter wie Durchsatz an Kohlenwasserstoffstrom, ausgedrückt als Raumgeschwindigkeit in der Einheit $[m^3/m^3{*}h]$, bezogen auf das Katalysatorvolumen, Temperatur und Druck werden analog zu denen bekannter Verfahren gewählt. Die Temperatur liegt üblicherweise im Bereich von 0 °C bis 180 °C und der Druck im Bereich von 2 bis 50 bar

Die Menge des eingesetzten Wasserstoffs, bezogen auf die Menge des zugeführten Kohlenwasserstoffstroms, ist abhängig von dem Gehalt des Kohlenwasserstoffstroms an unerwünschten ungesättigten Verbindungen und deren Art. Im Allgemeinen wird der Wasserstoff in einer Menge im Bereich vom 0,8 bis zum 5-fachen der stöchiometrisch zum vollständigen Wasserstoffumsatz beim Reaktordurchgang erforderlichen Menge zugegeben, vorzugsweise im Bereich vom 0,95 bis 2-fachen dieser Menge. Die Hydrierung von Dreifachbindungen läuft im Normalfall schneller ab als die konjugierter Doppelbindungen, und diese wiederum schneller als die unkonjugierter Doppelbindungen. Dies erlaubt eine entsprechende Steuerung des Verfahrens anhand der zugegebenen Wasserstoffmenge. In Sonderfällen, beispielsweise wenn eine hohe Isomerisierung von 1-Buten zu cis- oder trans-2-Buten gewünscht ist, kann bekanntlich auch ein höherer Wasserstoffüberschuß, beispielsweise ein zehnfacher Wasserstoffüberschuß verwendet werden. Der Wasserstoff kann Inerte enthalten, beispielsweise Edelgase wie Helium, Neon oder Argon, andere inerte Gase wie Stickstoff, Kohlendioxid und/oder niedere Alkane, etwa Methan, Ethan, Propan und/oder Butan. Solche Inertgase im Wasserstoff liegen vorzugsweise in einer Konzentration von weniger als 30 Vol.-% vor. Bevorzugterweise ist der Wasserstoff frei von Kohlenmonoxid.

Die Verfahren können in einem oder in mehreren parallelen oder hintereinandergeschalteten Reaktoren, jeweils im einfachen Durchgang oder in Kreislauffahrweise durchgeführt werden. Bei Durchführung der Verfahren in der Gas-/Flüssigphase wird der Kohlenwasserstoffstrom nach Durchtritt durch einen Reaktor üblicherweise in einem Abscheider von Gasen befreit und ein Teil der erhaltenen Flüssigkeit in den Reaktor zurückgeführt. Das Verhältnis zwischen zurückgeführtem und erstmals in den Reaktor eingespeistem Kohlenwasserstoffstrom, das sogenannte Rücklaufverhältnis, wird so eingestellt, dass unter den sonstigen Reaktionsbedingungen, wie Druck, Temperatur, Durchsatz und Wasserstoffmenge, der gewünschte Umsatz erreicht wird.

Einsatzzwecke der erfindungsgemäßen Verfahren sind etwa die Hydrierung von Alkinenen zu Alkadienen, von Alkinen, Alkinenen und Alkadienen zu Alkenen, von Phenylalkinen zu Phenylalkenen und/oder von Phenylalkenen zu Phenylalkanen.

Beispiele erfindungsgemäßer Verfahren sind die:

- zur selektiven Hydrierung von Acetylen in C2-Strömen zu Ethylen bei minimaler Bildung von Ethan (zur Vereinfachung im folgenden als "Verfahren A" bezeichnet),

- zur selektiven Hydrierung von Propin und/oder Propadien in C3-Strömen zu Propylen bei minimaler Bildung von Propan ("Verfahren B"),

- zur selektiven Hydrierung von 1-Butin, 2-Butin, 1,2-Butadien und/oder Vinylacetylen in C4-Strömen zu 1,3-Butadien, 1-Buten, cis- und/oder trans-2-Buten ("Verfahren C"),

- zur selektiven Hydrierung von 1-Butin, 2-Butin, 1,2-Butadien, 1,3-Butadien und/oder Vinylacetylen in C4-Strömen zu 1-Buten, cis- und/oder trans-2-Buten, bei butadienreichen C4-Strömen ("Roh-C4-Schnitt") oder butadienarmen C4-Strömen ("Raffinat I") ("Verfahren D"), und

- zur selektiven Hydrierung ungesättigter Verbindungen und/oder ungesättiger Substituenten aromatischer Verbindungen in C5+-Strömen zu höher gesättigten Verbindungen und/oder aromatischen Verbindungen mit höher gesättigten Substituenten bei minimaler Hydrierung der aromatischen Kerne ("Verfahren E"),

jeweils unter Verwendung des erfindungsgemäßen Katalysators.

Verfahren A wird üblicherweise als Gasphasenverfahren mit einer Raumgeschwindigkeit des gasförmigen C2-Stroms von 500 $m^3/m^3*h$, bezogen auf das Katalysatorvolumen, bis 10 000 $m^3/m^3*h$ bei einer Temperatur von 0 °C bis 250 °C und einem Druck von 0,01 bar bis 50 bar durchgeführt, wobei je Mol Acetylen im C2-Strom ein Mol Wasserstoff zugegeben wird.

Verfahren B wird üblicherweise als Gasphasenverfahren oder als Gas-/Flüssigphasenverfahren mit einer Raumgeschwindigkeit des flüssigen C3-Stroms von 1 $m^3/m^3*h$, bezogen auf das Katalysatorvolumen, bis 50 $m^3/m^3*h$ bei einer Temperatur von 0 °C bis 180 °C und einem Druck von 0,01 bar bis 50 bar durchgeführt, wobei je Mol Propin und Propadien im C3-Strom ein bis zwei Mole Wasserstoff zugegeben werden.

Verfahren C wird üblicherweise als Gas/Flüssigphasenverfahren mit einer Raumgeschwindigkeit des flüssigen C4-Stroms von 1 $m^3/m^3*h$, bezogen auf das Katalysatorvolumen, bis 50 $m^3/m^3*h$ bei einer Temperatur von 0 °C bis 180 °C und einem Druck von 2 bar bis 50 bar durchgeführt, wobei je Mol Butin, 1,2-Butadien und Vinylacetylen im C4-Strom ein bis zwei Mole Wasserstoff zugegeben werden. Verfahren C kann beispielsweise als selektive sogenannte "front end-Vinylacetylenhydrierung" vor einer Butadienextraktion eingesetzt werden.

Verfahren D wird üblicherweise als ein- oder zweistufiges Gas-/Flüssigphasenverfahren mit einer Raumgeschwindigkeit des flüssigen C4-Stroms im Bereich von 0,1 $m^3/m^3*h$, bezogen auf das Katalysatorvolumen, bis 60 $m^3/m^3*h$, vorzugsweise von 1 $m^3/m^3*h$ bis 50 $m^3/m^3*h$, bei einer Reaktoreingangstemperatur im Bereich von 20 °C bis 90 °C, vorzugsweise von 20 °C bis 70 °C, und einem Druck im Bereich von 5 bar bis 50 bar, vorzugsweise von 10 bar bis 30 bar durchgeführt, wobei je Mol Butin, Butadien und Vinylacetylen im C4-Strom ein Mol Wasserstoff zugegeben wird. Beispielsweise wird das Verfahren zweistufig durchgeführt, wobei der Butadiengehalt, der in typischen C4-Strömen aus Steamcrackern im Bereich von 20 Gew.-% bis 80 Gew.-%, bezogen auf den Gesamtstrom, liegt, in der ersten Stufe bis auf einen Gehalt im Bereich von 0,1 Gew.-% bis 20 Gew.-% und in der zweiten Stufe bis auf den gewünschten Restgehalt im Bereich von wenigen Gew.-ppm bis zu etwa 1 Gew.-% verringert wird. Es ist ebenso möglich, die Gesamtreaktion auf mehr als zwei Reaktoren, beispielsweise drei oder vier, zu verteilen. Die einzelnen Reaktionsstufen können unter teilweiser Rückführung des Kohlenwasserstoffstroms betrieben werden, das Rücklaufverhältnis liegt üblicherweise im Bereich von 0 bis 30. Isobuten bleibt bei der Durchführung von Verfahren D im wesentlichen unverändert erhalten und kann vor oder nach der Durchführung von Verfahren D mit bekannten Methoden aus dem C4-Strom abgetrennt werden. Verfahren D kann beispielsweise als Butadienhydrierung im C4-Strom (wenn Butadien nicht als Wertprodukt gewonnen werden soll) oder als selektive sogenannte "tail end-Vinylacetylenhydrierung" nach der Butadienextraktion aus dem C4-Strom verwendet werden.

Verfahren E wird vorzugsweise als Gas/Flüssigphasenverfahren mit einer Raumgeschwindigkeit des flüssigen C5+-Stroms von 0,5 $m^3/m^3*h$, bezogen auf das Katalysatorvolumen, bis 30 $m^3/m^3*h$ bei einer Temperatur von 0 °C bis 180 °C und einem Druck von 2 bar bis 50 bar durchgeführt, wobei je Mol zu hydrierender Bindung im C5+-Strom ein bis zwei Mole Wasserstoff zugegeben werden. Verfahren E kann beispielsweise als selektive Pyrolysebenzinhydrierung, als selektive Hydrierung von Olefinen in Reformatströmen oder Koksöfen-Kondensaten, zur Hydrierung von Phenylacetylen zu Styrol oder zur Hydrierung von Styrol zu Ethylbenzol eingesetzt werden.

**Beispiele**

Alle angegebenen Röntgenbeugungsdaten wurden mit einem Diffraktometer der Firma Siemens, Typ D 5000, unter Verwendung von Cu-$K_\alpha$-Strahlung gemessen. Der Meßbereich für 2θ war 10° bis 70°, entsprechend einem Bereich für die Netzebenenabstände von $5 \cdot 10^{-10}$ m bis $1,35 \cdot 10^{-10}$ m. Die Genauigkeit der erhaltenen Werte für die Netzebenenabstände beträgt $\pm\ 0,02 \cdot 10^{-10}$ m.

Umsatz U bezüglich Butadien, Butenin und Butin, Selektivitäten und 1-Buten-Erhalt (ein Maß für die Isomerisierungsaktivität des Katalysators) sind wie folgt definiert:

$$\begin{aligned} U = \ & [x_E(1,3\text{-Butadien}) + x_E(1,2\text{-Butadien}) + x_E(1\text{-Butin}) + x_E(\text{Butenin}) \\ & - x_P(1,3\text{-Butadien}) - x_P(1,2\text{-Butadien}) - x_P(1\text{-Butin}) - x_P(\text{Butenin})] \ / \\ & [x_E(1,3\text{-Butadien}) + x_E(1,2\text{-Butadien}) + x_E(1\text{-Butin}) + x_E(\text{Butenin})] \end{aligned}$$

$$\begin{aligned} \text{Gesamt-Buten-Selektivität } S_{GB} = \ & \\ 1 - \{[x_P(n\text{-Butan}) - x_E(n\text{-Butan})] \ / \ & [x_E(1,3\text{-Butadien}) + x_E(1,2\text{-Buta-} \\ & \text{dien}) + x_E(1\text{-Butin}) + x_E(\text{Butenin}) - x_P(1,3\text{-Butadien}) - x_P(1,2\text{-Buta-} \\ & \text{dien}) - x_P(1\text{-Butin}) - x_P(\text{Butenin})]\} \end{aligned}$$

$$\begin{aligned} \text{1-Buten-Selektivität } S_{1B} = \ & \\ [x_P(1\text{-Buten}) - x_E(1\text{-Buten})] \ / \ & [x_E(1,3\text{-Butadien}) + x_E(1,2\text{-Buta-} \\ & \text{dien}) + x_E(1\text{-Butin}) + x_E(\text{Butenin}) - x_P(1,3\text{-Butadien}) - x_P(1,2\text{-Buta-} \\ & \text{dien}) - x_P(1\text{-Butin}) - x_P(\text{Butenin})] \end{aligned}$$

$$\text{1-Buten-Erhalt } E_{1B} = 1 + \{[x_P(1\text{-Buten}) - x_E(1\text{-Buten})] \ / \ [x_E(1\text{-Buten})]\},$$

wobei
$x_E(A)$ der Massenanteil der Komponente A im Einsatzstoff und
$x_P(A)$ der Massenanteil der Komponente A im Produkt sind.

Vergleichsbeispiele und Beispiele 1 bis 8: Katalysator und Verfahren zur Flüssigphasenhydrierung von Roh-C4-Schnitt aus einem Steamcracker (Verfahren D, butadienreicher C4-Strom)

Vergleichsbeispiel 1: Herstellung von Vergleichskatalysator 1

In einem Mischer wurde Boehmit (Versal® 250, bezogen von Euro Support, Amsterdam) mit Wasser angefeuchtet, in einem Kollergang intensiv bearbeitet, bis die Masse gut verformbar war, und anschließend zu 3 mm-Strängen extrudiert. Danach wurden die Stränge 2 Stunden bei 120°C getrocknet und 4 Stunden bei 1200°C kalziniert. Danach wurden die Stränge mit $HNO_3$-saurer $Pd(NO_3)_2$-Lösung (pH = 1,3) nach der incipient wetness-Methode getränkt. Anschließend wurde der getränkte Träger 12 Stunden bei 120 °C getrocknet und 6 Stunden bei 330 °C kalziniert. Der Palladiumgehalt des fertigen Katalysators betrug 0,3 Gew.-% und sein Rüttelgewicht 1150 g/l. Verglichen mit dem erfindungsgemäßen Katalysator wurde der Träger von Vergleichskatalysator 1 zu lange und bei zu hohen Temperaturen kalziniert.

Der Katalysator zeigte folgendes Röntgenbeugungsmuster (nur Linien mit $I/I_o \geq 5\%$ sind angegeben):

| Netzebenenabstand d [$10^{-10}$ m] | relative Intensität $I/I_o$ |
|---|---|
| 3,48 | 0,45 |

Tabelle fortgesetzt

| Netzebenenabstand d [$10^{-10}$ m] | relative Intensität I/I$_o$ |
|---|---|
| 2,55 | 0,83 |
| 2,38 | 0,34 |
| 2,08 | 1 |
| 1,74 | 0,46 |
| 1,60 | 0,99 |
| 1,51 | 0,09 |
| 1,40 | 0,37 |
| 1,38 | 0,52 |

Vergleichsbeispiel 2: Herstellung von Vergleichskatalysator 2

In einem Mischer wurden 70 Gew.-% Boehmit (Versal® 250, bezogen von Euro Support, Amsterdam) und 30 Gew.-% α-Al$_2$O$_3$-Pulver (Typ CT 3000 SG der Firma Alcoa) mit Wasser angefeuchtet, in einem Kollergang intensiv bearbeitet, bis die Masse gut verformbar war, und anschließend zu 3 mm-Strängen extrudiert. Anschließend wurden die Stränge 2 Stunden bei 120°C getrocknet und 2 Stunden bei 900 °C kalziniert. Danach wurden die Stränge mit salpetersaurer wäßriger Pd(NO$_3$)$_2$-Lösung (pH = 0,2) nach der incipient wetness-Methode getränkt. Anschließend wurde der getränkte Träger 12 Stunden bei 120 °C getrocknet und 6 Stunden bei 330 °C kalziniert. Der Palladiumgehalt des fertigen Katalysators betrug 0,3 Gew.-% und sein Rüttelgewicht 890 g/l. Verglichen mit dem erfindungsgemäßen Katalysator wurde der Träger von Vergleichskatalysator 2 zu kurz bei zu niedrigen Temperaturen kalziniert.
Der Katalysator zeigte folgendes Röntgenbeugungsmuster (nur Linien mit I/Io ≥ 5% sind angegeben):

| Netzebenenabstand d [$10^{-10}$ m] | relative Intensität I/I$_o$ |
|---|---|
| 3,48 | 0,48 |
| 2,84 | 0,06 |
| 2,72 | 0,08 |
| 2,55 | 0,86 |
| 2,44 | 0,09 |
| 2,38 | 0,43 |
| 2,31 | 0,07 |
| 2,28 | 0,07 |
| 2,09 | 1 |
| 2,02 | 0,08 |
| 1,99 | 0,10 |
| 1,97 | 0,08 |
| 1,74 | 0,49 |
| 1,60 | 0,96 |
| 1,55 | 0,08 |
| 1,54 | 0,06 |
| 1,52 | 0,10 |
| 1,51 | 0,14 |
| 1,40 | 0,50 |

Tabelle fortgesetzt

| Netzebenenabstand d [$10^{-10}$ m] | relative Intensität $I/I_o$ |
|---|---|
| 1,39 | 0,19 |
| 1,37 | 0,62 |

Vergleichsbeispiel 3: Herstellung von Vergleichskatalysator 3

Ein kommerziell erhältlicher $Al_2O_3$-Träger (Spheralite 508F von Rhône-Poulenc) wurde mit salpetersaurer wäßriger $Pd(NO_3)_2$-Lösung (pH = 0,2) nach der incipient wetness-Methode getränkt. Anschlie-Bend wurde der getränkte Träger 12 Stunden bei 120 °C getrocknet und 6 Stunden bei 330 °C kalziniert. Der Palladiumgehalt des fertigen Katalysators betrug 0,3 Gew.-% und sein Rüttelgewicht 640 g/l. Vergleichskatalysator 3 entspricht etwa den aus DE-A 20 59 978 bekannten Katalysatoren.

Der Katalysator zeigte folgendes Röntgenbeugungsmuster (nur Linien mit $I/I_o \geq 5\%$ sind angegeben):

| Netzebenenabstand d [$10^{-10}$ m] | relative Intensität $I/I_o$ |
|---|---|
| 4,55 | 0,07 |
| 2,73 | 0,25 |
| 2,43 | 0,35 |
| 2,28 | 0,35 |
| 1,99 | 0,63 |
| 1,95 | 0,40 |
| 1,79 | 0,10 |
| 1,53 | 0,21 |
| 1,41 | 0,67 |
| 1,39 | 1 |

Beispiel 4 (erfindungsgemäß): Herstellung von Katalysator 4

In einem Mischer wurde Boehmit (Versal® 250, bezogen von Euro Support, Amsterdam) mit Wasser angefeuchtet, in einem Kollergang intensiv bearbeitet, bis die Masse gut verformbar war, und anschließend zu 3 mm-Strängen extrudiert. Danach wurden die Stränge 2 Stunden bei 120°C getrocknet und 2 Stunden bei 1000°C kalziniert. Danach wurden die Stränge mit $HNO_3$-saurer $Pd(NO_3)_2$-Lösung (pH = 0,5) nach der incipient wetness-Methode getränkt. Anschlie-Bend wurde der getränkte Träger 12 Stunden bei 120 °C getrocknet und 6 Stunden bei 330 °C kalziniert. Der Palladiumgehalt des fertigen Katalysators betrug 0,3 Gew.-% und sein Rüttelgewicht 620 g/l.

Der Katalysator zeigte folgendes Röntgenbeugungsmuster (nur Linien mit $I/I_o \geq 5\%$ sind angegeben):

| Netzebenenabstand d [$10^{-10}$ m] | relative Intensität $I/I_o$ |
|---|---|
| 5,47 | 0,05 |
| 4,54 | 0,10 |
| 3,48 | 0,27 |
| 2,85 | 0.38 |
| 2,73 | 0,68 |
| 2,55 | 0,62 |
| 2,44 | 0,47 |
| 2,38 | 0,39 |
| 2,31 | 0,39 |

Tabelle fortgesetzt

| Netzebenenabstand d [$10^{-10}$ m] | relative Intensität $I/I_o$ |
|---|---|
| 2,26 | 0,35 |
| 2,09 | 0,62 |
| 2,02 | 0,48 |
| 1,91 | 0,33 |
| 1,80 | 0,15 |
| 1,74 | 0,33 |
| 1,60 | 0,56 |
| 1,54 | 0,28 |
| 1,51 | 0,20 |
| 1,49 | 0,23 |
| 1,45 | 0,32 |
| 1,40 | 0,71 |
| 1,39 | 1 |
| 1,38 | 0,49 |

Durchführung der Versuche zur Flüssigphasenhydrierung von Roh-C4-Schnitt aus einem Steamcracker

Die Versuche wurden in einer Versuchsanlage durchgeführt, die mit einem elektrisch beheizbaren Festbettreaktor von 16 mm Durchmesser und 2 m Länge, einem Vorheizer, einem Abscheider, einem Kühler für den Reaktoraustrag und einem Flüssigkeitskreislauf ausgestattet war. Die verwendete Katalyatormenge war 200 ml. Der Roh-C4-Schnitt wurde über eine Förderpumpe zudosiert und an einer Mischstelle mit dem mengengeregelt zugeführten Wasserstoff vermischt. Im Abscheider wurde der Reaktoraustrag in Gas- und Flüssigphase getrennt, die Gasphase wurde verworfen. Der Großteil der Flüssigphase wurde im Kreislauf wieder in den Reaktor zurückgeführt. Ein kleinerer, der dem Reaktor erstmals zugeführten Menge des Roh-C4-Schnitts entsprechender Teil wurde kontinuierlich aus dem Abscheider als Produkt entnommen. Die Analysen wurden mittels eines Gaschromatographen durchgeführt.

Vor der erstmaligen Zufuhr von Kohlenwasserstoff in den Reaktor wurden die Katalysatoren über 12 Stunden bei 120 °C und 5 bar Druck mit Wasserstoff behandelt. Anschließend wurde die Anlage mit bereits selektiv hydriertem C4-Schnitt gefüllt, auf 60 °C beheizt und in Betrieb genommen, nach Erreichen der Betriebsbedingungen (Druck, Temperatur, Durchsatz) wurden Roh-C4-Schnitt und Wasserstoff zugeführt.

Der Durchsatz durch den Reaktor, ausgedrückt als Raumzeitgeschwindigkeit des dem Reaktor erstmals zugeführten flüssigen Kohlenwasserstoffstroms ("Frisch-Feed"), betrug 9,0 m$^3$/m$^3$h, die Temperatur des Frisch-Feeds wurde mit dem Vorheizer auf 60 °C eingestellt. Das Rücklaufverhältnis wurde so eingestellt, dass der Temperaturanstieg im Reaktor zwischen 20 und 50 °C betrug. Die jeweils eingestellten Rücklaufverhältnisse sind unten angegeben.
Der Druck betrug 15±1 bar, das molare Verhältnis von zugegebenem Wasserstoff zu im Roh-C4-Schnitt vorhandenem Butadien wurde auf 1,00 bis 1,02 eingestellt.

Vergleichsbeispiel 5: Hydrierung mit Vergleichskatalysator 1

Das Rücklaufverhältnis betrug 8,2, die Zusammensetzungen von Roh-C4-Schnitt und hydriertem Produkt waren:

| | C4-Schnitt | Produkt |
|---|---|---|
| Butadien + Butenin + Butin [Gew.-%] | 43,7 | 1,1 |
| 1-Buten [Gew.-%] | 14,3 | 36,3 |
| trans-2-Buten [Gew.-%] | 4,5 | 20,3 |
| cis-2-Buten [Gew.-%] | 3,3 | 7,3 |

Tabelle fortgesetzt

|  | C4-Schnitt | Produkt |
|---|---|---|
| i-Buten [Gew.-%] | 23,7 | 23,8 |
| i-Butan [Gew.-%] | 3,0 | 3,0 |
| n-Butan [Gew.-%] | 7,2 | 7,8 |
| C5-Kohlenwasserstoffe [Gew.-%] | 0,3 | 0,4 |

Vergleichsbeispiel 6: Hydrierung mit Vergleichskatalysator 2

Das Rücklaufverhältnis betrug 11, die Zusammensetzungen von Roh-C4-Schnitt und hydriertem Produkt waren:

|  | C4-Schnitt | Produkt |
|---|---|---|
| Butadien + Butenin + Butin [Gew.-%] | 43,2 | 1,3 |
| 1-Buten [Gew.-%] | 14,4 | 38,4 |
| trans-2-Buten [Gew.-%] | 4,4 | 19,0 |
| cis-2-Buten [Gew.-%] | 3,0 | 5,7 |
| i-Buten [Gew.-%] | 23,9 | 23,8 |
| i-Butan [Gew.-%] | 3,0 | 3,1 |
| n-Butan [Gew.-%] | 8,0 | 8,6 |
| C5-Kohlenwasserstoffe [Gew.-%] | 0,1 | 0,1 |

Vergleichsbeispiel 7: Hydrierung mit Vergleichskatalysator 3

Das Rücklaufverhältnis betrug 8,2, die Zusammensetzungen von Roh-C4-Schnitt und hydriertem Produkt waren:

|  | C4-Schnitt | Produkt |
|---|---|---|
| Butadien + Butenin + Butin [Gew.-%] | 43,7 | 1,5 |
| 1-Buten [Gew.-%] | 14,3 | 38,4 |
| trans-2-Buten [Gew.-%] | 4,5 | 19,7 |
| cis-2-Buten [Gew.-%] | 3,3 | 6,1 |
| i-Buten [Gew.-%] | 23,6 | 23,6 |
| i-Butan [Gew.-%] | 2,9 | 2,9 |
| n-Butan [Gew.-%] | 7,2 | 7,4 |
| C5-Kohlenwasserstoffe [Gew.-%] | 0,5 | 0,4 |

Beispiel 8: Hydrierung mit Katalysator 4

Das Rücklaufverhältnis betrug 8,2, die Zusammensetzungen von Roh-C4-Schnitt und hydriertem Produkt waren:

|  | C4-Schnitt | Produkt |
|---|---|---|
| Butadien+Butenin+Butin [Gew.-%] | 46,3 | 1,5 |
| 1-Buten [Gew.-%] | 15,3 | 41,6 |

Tabelle fortgesetzt

|  | C4-Schnitt | Produkt |
|---|---|---|
| trans-2-Buten [Gew.-%] | 5,1 | 20,4 |
| cis-2-Buten [Gew.-%] | 3,8 | 6,6 |
| i-Buten [Gew.-%] | 23,9 | 23,9 |
| i-Butan [Gew.-%] | 1,0 | 1,0 |
| n-Butan [Gew.-%] | 4,4 | 4,5 |
| C5-Kohlenwasserstoffe [Gew.-%] | 0,2 | 0,5 |

Diskussion der Vergleichsbeispiele und Beispiele 1-8

Die in den Hydrierversuchen erreichten Umsätze und Selektivitäten berechnen sich zu den folgenden Werten, in Mol.-%:

|  | U | $S_{GB}$ | $S_{1B}$ |
|---|---|---|---|
| Vergleichskatalysator 1 | 97,5 | 98,6 | 51,6 |
| Vergleichskatalysator 2 | 97,0 | 98,6 | 57,3 |
| Vergleichskatalysator 3 | 96,6 | 99,5 | 57,1 |
| Katalysator 4 | 96,8 | 99,8 | 58,7 |

Vergleichskatalysator 1 zeigt eine unbefriedigende Gesamt-Buten-Selektivität und die mit deutlichem Abstand niedrigste und völlig unbefriedigende 1-Buten-Selektivität. Vergleichskatalysator 2 zeigt bei minimal niedrigerem Umsatz zwar ebenfalls eine unbefriedigende Gesamt-Buten-Selektivität, aber eine signifikant höhere, wenn auch noch nicht befriedigende 1-Buten-Selektivität. Vergleichskatalysator 3 zeigt bei minimal niedrigerem Umsatz eine sehr deutlich verbesserte Gesamt-Buten-Selektivität bei gleicher 1-Buten-Selektivität. Der erfindungsgemäße Katalysator 4 zeigt bei vergleichbarem Umsatz eine nochmals verbesserte Gesamt-Buten-Selektivität und eine deutlich verbesserte 1-Buten-Selektivität. Mit dem erfindungsgemäßen Katalysator wird sowohl die unerwünschte Überhydrierung zu n-Butan am weitesten unterdrückt als auch das gewünschte Wertprodukt 1-Buten in der besten Ausbeute erhalten.

Vergleichsbeispiele und Beispiele 9 bis 14: Katalysator und Verfahren zur Flüssigphasenhydrierung eines C4-Stroms aus einem Steamcracker nach einer Butadienextraktion (Verfahren D, butadienarmer C4-Strom)

Vergleichsbeispiel 9: Herstellung von Vergleichskatalysator 5

Beispiel 1 aus EP-A 653243 wurde nachgearbeitet, wobei jedoch ein Palladiumgehalt von 0,3 Gew.-% eingestellt wurde. Der Katalysator hatte ein Rüttelgewicht von 380 g/l.

Der Katalysator zeigte folgendes Röntgenbeugungsmuster (nur Linien mit I/Io ≥5% sind angegeben):

| Netzebenenabstand d [$10^{-10}$ m] | relative Intensität $I/I_o$ |
|---|---|
| 2,64 | 0,43 |
| 2,42 | 0,56 |
| 2,29 | 0,50 |
| 1,97 | 0,67 |
| 1,52 | 0,34 |
| 1,40 | 1 |

Vergleichsbeispiel 10: Herstellung von Vergleichskatalysator 6

Ein kommerziell erhältlicher $Al_2O_3$-Träger (Spheralite 508F von Rhöne-Poulenc) wurde mit einer salpetersauren wäßrigen Lösung von $Pd(NO_3)_2$ und $AgNO_3$ (pH = 0,2) nach der incipient wetness-Methode getränkt. Anschließend wurde der getränkte Träger 12 Stunden bei 120 °C getrocknet und 6 Stunden bei 330 °C kalziniert. Der Palladiumgehalt des fertigen Katalysators betrug 0,2 Gew.-%, sein Silbergehalt 0,1 Gew.-% und sein Rüttelgewicht 640 g/1. Vergleichskatalysator 3 entspricht etwa den aus DE-A 31 19 850 bekannten Katalysatoren.

Der Katalysator zeigte folgendes Röntgenbeugungsmuster (nur Linien mit I/Io ≥ 5% sind angegeben):

| Netzebenenabstand d [$10^{-10}$ m] | relative Intensität I/I$_o$ |
| --- | --- |
| 4,50 | 0,06 |
| 2,74 | 0,23 |
| 2,43 | 0,37 |
| 2,28 | 0,35 |
| 1,99 | 0,64 |
| 1,95 | 0,42 |
| 1,79 | 0,11 |
| 1,52 | 0,24 |
| 1,40 | 0,76 |
| 1,39 | 1 |

Beispiel 11 (erfindungsgemäß): Herstellung von Katalysator 7

In einem Mischer wurde Boehmit (Versal® 250, bezogen von Euro Support, Amsterdam) mit Wasser angefeuchtet, in einem Kollergang intensiv bearbeitet, bis die Masse gut verformbar war, und anschließend zu 3 mm-Strängen extrudiert. Danach wurden die Stränge 2 Stunden bei 120 °C getrocknet und 2 Stunden bei 1000°C kalziniert. Danach wurden die Stränge mit einer wäßrigen salpetersauren Lösung von $Pd(NO_3)_2$ und $AgNO3$ (pH = 0,5) nach der incipient wetness-Methode getränkt. Anschließend wurde der getränkte Träger 12 Stunden bei 120 °C getrocknet und 6 Stunden bei 330 °C kalziniert. Der Palladiumgehalt des fertigen Katalysators betrug 0,2 Gew.-%, sein Silbergehalt 0,1 Gew.-% und sein Rüttelgewicht 620 g/l.

Der Katalysator zeigte folgendes Röntgenbeugungsmuster (nur Linien mit I/Io ≥ 5% sind angegeben):

| Netzebenenabstand d [$10^{-10}$ m] | relative Intensität I/I$_o$ |
| --- | --- |
| 5,47 | 0,05 |
| 4,54 | 0,10 |
| 3,48 | 0,27 |
| 2,85 | 0,38 |
| 2,73 | 0,68 |
| 2,55 | 0,62 |
| 2,44 | 0,47 |
| 2,38 | 0,39 |
| 2,31 | 0,39 |
| 2,26 | 0,35 |
| 2,09 | 0,62 |
| 2,02 | 0,48 |
| 1,91 | 0,33 |
| 1,80 | 0,15 |
| 1,74 | 0,33 |

Tabelle fortgesetzt

| Netzebenenabstand d [$10^{-10}$ m] | relative Intensität $I/I_o$ |
|---|---|
| 1,60 | 0,56 |
| 1,54 | 0,28 |
| 1,51 | 0,20 |
| 1,49 | 0,23 |
| 1,45 | 0,32 |
| 1,40 | 0,71 |
| 1,39 | 1 |
| 1,38 | 0,49 |

Durchführung der Versuche zur Flüssigphasenhydrierung von C4-Strom aus einem Steamcracker, nach Butadienextraktion

Die Versuche wurden in der vor Beispiel 5 beschriebenen, bereits für die Versuche zur Flüssigphasenhydrierung von Roh-C4-Schnitt verwendeten, Versuchsanlage auf die gleiche Weise wie die Versuche der Beispiele 5 bis 8 durchgeführt, mit den Ausnahmen, dass statt Roh-C4-Schnitt ein durch ein übliches Extraktionsverfahren von Butadien befreiter C4-Strom ("Raffinat I") eingesetzt wurde, dass die Raumzeitgeschwindigkeit 15 m³/m³h betrug, der Druck 12 bar (Ausnahme bei Beispiel 14: 9 bar), und dass das Verhältnis von Wasserstoff zu Butadien differierte, letzteres ist bei den einzelnen Beispielen angegeben. Dieses Verhältnis wurde so eingestellt, dass unter den sonstigen Reaktionsbedingungen ein Butadienumsatz von 99,8 %, entsprechend einem Rest-Butadiengehalt von 10 ppm, resultierte. Das Rücklaufverhältnis wurde in allen Fällen auf 1,0 eingestellt; es ist bei diesen Versuchen deutlich geringer als bei den Versuchen 5 bis 8, da deutlich weniger Hydrierwärme entsteht.

Vergleichsbeispiel 12: Hydrierung mit Vergleichskatalysator 5

Das molare Verhältnis von Wasserstoff zu Butadien im Raffinat I betrug 5,2, die Zusammensetzungen von Raffinat I und hydriertem Produkt waren:

| | Raffinat I | Produkt |
|---|---|---|
| Butadien + Butenin + Butin [Gew.-%] | 0,53 | 0,001 |
| 1-Buten [Gew.-%] | 27,0 | 11,0 |
| trans-2-Buten [Gew.-%] | 10,0 | 18,7 |
| cis-2-Buten [Gew.-%] | 5,2 | 11,2 |
| i-Buten [Gew.-%] | 42,8 | 42,6 |
| i-Butan [Gew.-%] | 3,1 | 3,0 |
| n-Butan [Gew.-%] | 11,0 | 13,2 |
| C5-Kohlenwasserstoffe [Gew.-%] | 0,4 | 0,3 |

Vergleichsbeispiel 13: Hydrierung mit Vergleichskatalysator 6

Das molare Verhältnis von Wasserstoff zu Butadien im Raffinat I betrug 2,9, die Zusammensetzungen von Raffinat I und hydriertem Produkt waren:

| | C4-Schnitt | Produkt |
|---|---|---|
| Butadien + Butenin + Butin [Gew.-%] | 0,43 | 0,001 |
| 1-Buten [Gew.-%] | 25,1 | 20,8 |

Tabelle fortgesetzt

|  | C4-Schnitt | Produkt |
|---|---|---|
| trans-2-Buten [Gew.-%] | 7,8 | 10,2 |
| cis-2-Buten [Gew.-%] | 5,4 | 7,2 |
| i-Buten [Gew.-%] | 42,3 | 42,2 |
| i-Butan [Gew.-%] | 4,8 | 4,6 |
| n-Butan [Gew.-%] | 14,0 | 14,8 |
| C5-Kohlenwasserstoffe [Gew.-%] | 0,2 | 0,2 |

Beispiel 14: Hydrierung mit Katalysator 7

Das molare Verhältnis von Wasserstoff zu Butadien im Raffinat I betrug 1,4, die Zusammensetzungen von Raffinat I und hydriertem Produkt waren:

|  | C4-Schnitt | Produkt |
|---|---|---|
| Butadien+Butenin+Butin [Gew.-%] | 0,55 | 0,001 |
| 1-Buten [Gew.-%] | 23,9 | 23,4 |
| trans-2-Buten [Gew.-%] | 8,1 | 8,7 |
| cis-2-Buten [Gew.-%] | 5,7 | 6,0 |
| i-Buten [Gew.-%] | 43,6 | 43,7 |
| i-Butan [Gew.-%] | 4,5 | 4,4 |
| n-Butan [Gew.-%] | 13,5 | 13,6 |
| C5-Kohlenwasserstoffe [Gew.-%] | 0,15 | 0,2 |

Diskussion der Vergleichsbeispiele und Beispiele 9-14

Die in den Hydrierversuchen erreichten Umsätze, Gesamt-Buten-Selektivitäten, Werte für den 1-Buten-Erhalt, in Mol.-%, und die Zunahme von n-Butan $Z_{nB}$, das Maß für die Überhydrierung, in Gew.-%, berechnen sich zu den folgenden Werten:

|  | U | $S_{GB}$ | $E_{1B}$ | $Z_{nB}$ |
|---|---|---|---|---|
| Vergleichskatalysator 5 | 99,8 | -310 | 40,6 | 2,2 |
| Vergleichskatalysator 6 | 99,8 | -91 | 82,8 | 0,8 |
| Katalysator 7 | 99,8 | 76 | 98,0 | 0,1 |

Vergleichskatalysator 5 zeigt einen vergleichsweise niedrigen 1-Buten-Erhalt und eine vergleichsweise hohe Überhydrierung zu n-Butan von 2,2 Gew.-%. Vergleichskatalysator 6 zeigt bei gleichem Umsatz einen bereits deutlich höheren, aber dennoch unbefriedigenden 1-Buten-Erhalt und eine deutlich verringerte, aber dennoch unbefriedigende Überhydrierung. Der erfindungsgemäße Katalysator 7 zeigt dagegen gute Selektivität und geringe Überhydrierung.

Beispiel 15: Verfahren zur Entfernung von Butadien aus Roh-C4-Schnitt durch selektive zweistufige Flüssigphasenhydrierung (Verfahren D, butadienreicher C4-Strom)

Roh-C4-Schnitt wurde in der vor Beispiel 5 beschriebenen Versuchsanlage und auf die dort beschriebene Weise, aber mit einer Raumzeitgeschwindigkeit von 9,0 m$^3$/m$^3$h, einem Rücklaufverhältnis von 8,2, bei einer Temperatur von 60 °C, einem Druck von 15 bar und bei einem molaren Verhältnis von Wasserstoff zu im Roh-C4-Strom enthaltenen Butadien von 1,00 unter Verwendung von Katalysator 4 hydriert. Das Produkt wurde anschließend in einer weiteren Anlage, die sich von der ersten durch die fehlende Rückführung unterschied, auf dieselbe Weise, aber mit einer Raum-zeitgeschwindigkeit von 15 $m^3/m^3h$, bei einer Temperatur von 60 °C, einem Druck von 9 bar und bei einem molaren Verhältnis von Wasserstoff zu im Produkt der ersten Stufe enthaltenen Butadien von 1,4 unter Verwendung von Katalysator 7 hydriert.

Die Zusammensetzungen von Roh-C4-Schnitt und Produkten waren:

| [Gew.-%] | C4-Schnitt | 1. Stufe | 2. Stufe |
|---|---|---|---|
| Butadien+Butenin+Butin | 46,3 | 0,48 | 0,001 |
| 1-Buten | 15,3 | 39,5 | 38,7 |
| trans-2-Buten | 5,1 | 22,4 | 23,0 |
| cis-2-Buten | 3,8 | 7,7 | 8,3 |
| i-Buten | 23,9 | 23,9 | 23,9 |
| i-Butan | 1,0 | 1,0 | 1,0 |
| n-Butan | 4,4 | 4,7 | 4,8 |
| C5-Kohlenwasserstoffe | 0,2 | 0,3 | 0,3 |

Über beide Stufen wurde ein Umsatz U von 99,8 %, eine Selektivität $S_{GB}$ von 99,1 % und eine Selektivität $S_{1B}$ von 50,5 bei einer n-Butan-Bildung $Z_{nB}$ von 0,4 % erreicht.

**Patentansprüche**

1. Katalysator, der mindestens ein hydrieraktives Metall auf einem Aluminiumoxidträger umfaßt und ungebraucht im Röntgendiffraktogramm Reflexe zeigt, die den folgenden Netzebenenabständen entsprechen:

| Netzebenenabstand d [$10^{-10}$ m] | relative Intensität $I/I_o$ |
|---|---|
| 4,52 | 0,05 bis 0,1 |
| 2,85 | 0,35 bis 0,45 |
| 2,73 | 0,65 bis 0,8 |
| 2,44 | 0,45 bis 0,55 |
| 2,31 | 0,35 bis 0,45 |
| 2,26 | 0,35 bis 0,45 |
| 2,02 | 0,45 bis 0,6 |
| 1,91 | 0,3 bis 0,4 |
| 1,80 | 0,1 bis 0,25 |
| 1,54 | 0,25 bis 0,35 |
| 1,51 | 0 bis 0,35 |
| 1,49 | 0,2 bis 0,3 |
| 1,45 | 0,25 bis 0,35 |
| 1,39 | 1 |

2. Katalysator nach Anspruch 1, der ungebraucht im Röntgendiffraktogramm mindestens einen zusätzlichen Reflex zeigt, der einem der folgenden Netzebenenabstände [in $10^{-10}$ m] entspricht: 3,48, 2,55, 2,38, 2,09, 1,78, 1,74, 1,62, 1,60, 1,57, 1,42, 1,40 und 1,37.

3. Katalysator nach den Ansprüchen 1 oder 2, wobei das hydrieraktive Metall oder die hydrieraktiven Metalle aus der 8., 9. oder 10. Gruppe des Periodensystems der Elemente stammen.

4. Katalysator nach den Ansprüchen 1 bis 3, wobei das hydrieraktive Metall Platin und/oder Palladium ist.

5. Katalysator nach Anspruch 4, wobei das hydrieraktive Metall Palladium ist und in einer Menge von mindestens 0,05 Gew.-% und höchstens 2 Gew.-%, bezogen auf das Gesamtgewicht des Katalysators, enthalten ist.

6. Katalysator nach den Ansprüchen 1 bis 5, wobei der Katalysator zusätzlich zum hydrieraktiven Metall mindestens ein Metall der 11. Gruppe des Periodensystems der Elemente umfaßt.

7. Katalysator nach Anspruch 6, wobei das Metall der 11. Gruppe des Periodensystems der Elemente Kupfer und/ oder Silber ist.

8. Katalysator nach Anspruch 7, wobei das Metall der 11. Gruppe Silber ist und in einer Menge von mindestens 0,01 Gew.-% und höchstens 1 Gew.-%, bezogen auf das Gesamtgewicht des Katalysators, enthalten ist.

9. Verfahren zur Herstellung eines in den Ansprüchen 1 bis 8 beschriebenen Katalysators durch Behandeln eines aluminiumhaltigen Rohstoffs mit Wasser, verdünnter Säure oder verdünnter Base, Verformung zu Formkörpern, Trocknung der Formkörper, Kalzinierung der getrockneten Formkörper, Tränkung der kalzinierten Formkörper mit einer die abzuscheidenden Metalle enthaltenden Lösung, Trocknung der getränkten Formkörper und Fertigstellung des Katalysators durch Kalzinierung der getränkten und getrockneten Formkörper, **dadurch gekennzeichnet, daß** man die getrockneten Formkörper bei einer Temperatur oberhalb von 900 °C und unterhalb von 1100 °C kalziniert.

10. verfahren nach Anspruch 9, **dadurch gekennzeichnet, daß** man die getrockneten Formkörper über einen Zeitraum von mindestens 0,5 und höchstens 5 Stunden kalziniert.

11. Verwendung des in den Ansprüchen 1 bis 8 beschriebenen Katalysators zur Hydrierung ungesättigter Verbindungen.

12. Verwendung des in den Ansprüchen 1 bis 8 beschriebenen Katalysators zur selektiven Hydrierung von Alkinenen zu Alkadienen, zur selektiven Hydrierung von Alkinen, Alkinenen und Alkadienen zu Alkenen, zur selektiven Hydrierung von Phenylalkinen zu Phenylalkenen und/oder Phenylalkanen und/oder zur selektiven Hydrierung von Phenylalkenen zu Phenylalkanen.

13. Verfahren zur selektiven Hydrierung ungesättigter Verbindungen in Kohlenwasserstoffströmen in der Gas- oder Flüssigphase bei Temperaturen im Bereich von 0 °C bis 180 °C und Drücken im Bereich von 2 bis 50 bar, **dadurch gekennzeichnet, daß** man die selektive Hydrierung in einer oder mehreren Reaktionsstufen durchführt und in mindestens einer Reaktionsstufe den in den Ansprüchen 1 bis 8 beschriebenen Katalysator verwendet.

14. Verfahren nach Anspruch 13, **dadurch gekennzeichnet, daß** man Acetylen in einem C2-Strom selektiv zu Ethylen hydriert.

15. Verfahren nach Anspruch 13, **dadurch gekennzeichnet, daß** man Propin und/oder Propadien in einem C3-Strom selektiv zu Propylen hydriert.

16. Verfahren nach Anspruch 13, **dadurch gekennzeichnet, daß** man 1-Butin, 2-Butin, 1,2-Butadien und/oder Vinylacetylen in einem C4-Strom selektiv zu 1,3-Butadien, 1-Buten, cis- und/oder trans-2-Buten hydriert.

17. Verfahren nach Anspruch 13, **dadurch gekennzeichnet, daß** man 1-Butin, 2-Butin, 1,2-Butadien, 1,3-Butadien und/oder Vinylacetylen in einem C4-Strom selektiv zu 1-Buten, cis- und/oder trans-2-Buten hydriert.

18. Verfahren nach Anspruch 13, **dadurch gekennzeichnet, daß** man ungesättigte Verbindungen und/oder ungesättige Substituenten aromatischer Verbindungen in einem C5+-Strom selektiv zu höher gesättigten Verbindungen und/ oder aromatischen Verbindungen mit höher gesättigten Substituenten hydriert.

**Claims**

1. A catalyst which comprises at least one metal having hydrogenation activity on an alumina carrier and, in the unused state, shows reflections in the X-ray diffraction pattern which correspond to the following lattice plane spacings:

| Lattice plane spacing d [$10^{-10}$ m] | Relative intensity $I/I_o$ |
|---|---|
| 4.52 | 0.05 to 0.1 |
| 2.85 | 0.35 to 0.45 |
| 2.73 | 0.65 to 0.8 |
| 2.44 | 0.45 to 0.55 |
| 2.31 | 0.35 to 0.45 |
| 2.26 | 0.35 to 0.45 |
| 2.02 | 0.45 to 0.6 |
| 1.91 | 0.3 to 0.4 |
| 1.80 | 0.1 to 0.25 |
| 1.54 | 0.25 to 0.35 |
| 1.51 | 0 to 0.35 |
| 1.49 | 0.2 to 0.3 |
| 1.45 | 0.25 to 0.35 |
| 1.39 | 1 |

2. The catalyst according to claim 1, which, in the unused state, shows at least one additional reflection in the X-ray diffraction pattern which corresponds to one of the following lattice plane spacings [in $10^{-10}$ m]: 3.48, 2.55, 2.38, 2.09, 1.78, 1.74, 1.62, 1.60, 1.57, 1.42, 1.40 and 1.37.

3. The catalyst according to claim 1 or 2, the metal having hydrogenation activity or the metals having hydrogenation activity being a metal or metals from group 8, 9 or 10 of the Periodic Table of Elements.

4. The catalyst according to any of claims 1 to 3, the metal having hydrogenation activity being platinum and/or palladium.

5. The catalyst according to claim 4, the metal having hydrogenation activity being palladium and being comprised in an amount of at least 0.05% by weight and not more than 2% by weight, based on the total weight of the catalyst.

6. The catalyst according to any of claims 1 to 5, the catalyst comprising at least one metal of group 11 of the Periodic Table of Elements in addition to the metal having hydrogenation activity.

7. The catalyst according to claim 6, the metal of group 11 of the Periodic Table of Elements being copper and/or silver.

8. The catalyst according to claim 7, the metal of group 11 being silver and being comprised in an amount of at least 0.01% by weight and not more than 1% by weight, based on the total weight of the catalyst.

9. A process for the preparation of a catalyst described in any of claims 1 to 8 by treating an aluminum-containing raw material with water, dilute acid or dilute base, shaping to give moldings, drying the moldings, calcining the dried moldings, impregnating the calcined moldings with a solution comprising the metals to be deposited, drying the impregnated moldings and finishing the catalyst by calcining the impregnated and dried moldings, wherein the dried moldings are calcined at above 900°C and below 1100°C.

10. The process according to claim 9, wherein the dried moldings are calcined over a period of at least 0.5 hour and not more than 5 hours.

11. The use of the catalyst described in any of claims 1 to 8 for the hydrogenation of unsaturated compounds.

12. The use of the catalyst described in any of claims 1 to 8 for the selective hydrogenation of alkynenes to alkadienes, for the selective hdyrogenation of alkynes, alkynenes and alkadienes to alkenes, for the selective hdyrogenation of phenylalkynes to phenylalkenes and/or phenylalkanes and/or for the selective hydrogenation of phenylalkenes to phenylalkanes.

13. A process for the selective hydrogenation of unsaturated compounds in hydrocarbon streams in the gas or liquid phase at from 0°C to 180°C and from 2 to 50 bar, wherein the selective hydrogenation is carried out in one or more reaction stages and the catalyst described in any of claims 1 to 8 is used in at least one reaction stage.

14. The process according to claim 13, wherein acetylene in a C2 stream is hydrogenated selectively to ethylene.

15. The process according to claim 13, wherein propyne and/or propadiene in a C3 stream are hydrogenated selectively to propylene.

16. The process according to claim 13, wherein 1-butyne, 2-butyne, 1,2-butadiene and/or vinylacetylene in a C4 stream are hydrogenated selectively to 1,3-butadiene, 1-butene, cis-2-butene and/or trans-2-butene.

17. The process according to claim 13, wherein 1-butyne, 2-butyne, 1,2-butadiene, 1,3-butadiene and/or vinylacetylene in a C4 stream are hydrogenated selectively to 1-butene, cis-2-butene and/or trans-2-butene.

18. The process according to claim 13, wherein unsaturated compounds and/or unsaturated substituents of aromatic compounds in a C5+ stream are hydrogenated selectively to more highly saturated compounds and/or aromatic compounds having more highly saturated substituents.

**Revendications**

1. Catalyseur qui comporte au moins un métal actif d'hydrogénation sur un support d'oxyde d'aluminium et qui, non utilisé, montre dans un diffractogramme aux rayons X des reflets qui correspondent aux écartements de plans réticulaires suivants :

| Ecartement de plans réticulaires d [$10^{-10}$ m] | Intensité relative $I/I_o$ |
|---|---|
| 4,52 | 0,05 à 0,1 |
| 2,85 | 0,35 à 0,45 |
| 2,73 | 0,65 à 0,8 |
| 2,44 | 0,45 à 0,55 |
| 2,31 | 0,35 à 0,45 |
| 2,26 | 0,35 à 0,45 |
| 2,02 | 0,45 à 0,6 |
| 1,91 | 0,3 à 0,4 |
| 1,80 | 0,1 à 0,25 |
| 1,54 | 0,25 à 0,35 |
| 1,51 | 0 à 0,35 |
| 1,49 | 0,2 à 0,3 |
| 1,45 | 0,25 à 0,35 |
| 1,39 | 1 |

2. Catalyseur suivant la revendication 1, qui, non utilisé, montre dans un diffractogramme aux rayons X au moins un reflet additionnel qui correspond à un des écartements de plans réticulaires suivants [en 10⁻¹⁰ m] : 3,48, 2,55, 2,38, 2,09, 1,78, 1,74, 1,62, 1,60, 1,57, 1,42, 1,40 et 1,37.

3. Catalyseur suivant les revendications 1 ou 2, dans lequel le métal actif d'hydrogénation ou les métaux actifs d'hydrogénation proviennent des groupes 8, 9 ou 10 du système périodique des éléments.

4. Catalyseur suivant les revendications 1 à 3, dans lequel le métal actif d'hydrogénation est du platine et/ou du palladium.

5. Catalyseur suivant la revendication 4, dans lequel le métal actif d'hydrogénation est du palladium et est contenu en une quantité d'au moins 0,05 % en poids et d'au plus 2 % en poids, par rapport au poids total du catalyseur.

6. Catalyseur suivant les revendications 1 à 5, dans lequel le catalyseur comporte en supplément au métal actif d'hydrogénation au moins un métal du groupe 11 du système périodique des éléments.

7. Catalyseur suivant la revendication 6, dans lequel le métal du groupe 11 du système périodique des éléments est du cuivre et/ou de l'argent.

8. Catalyseur suivant la revendication 7, dans lequel le métal du groupe 11 est de l'argent et est contenu en une quantité d'au moins 0,01 % en poids et d'au plus 1 % en poids, par rapport au poids total du catalyseur.

9. Procédé de préparation d'un catalyseur décrit dans les revendications 1 à 8, par traitement d'une matière première contenant de l'aluminium avec de l'eau, un acide dilué ou une base diluée, formage en corps façonnés, séchage des corps façonnés, calcination des corps façonnés séchés, imprégnation des corps façonnés calcinés par une solution contenant les métaux à déposer, séchage des corps façonnés imprégnés et finition du catalyseur par calcination des corps façonnés imprégnés et séchés, **caractérisé en ce qu'**on calcine les corps façonnés séchés à une température supérieure à 900°C et inférieure à 1100°C.

10. Procédé suivant la revendication 9, **caractérisé en ce qu'**on calcine les corps façonnés séchés pendant un intervalle de temps d'au moins 0,5 à au plus 5 heures.

11. Utilisation du catalyseur décrit dans les revendications 1 à 8, pour l'hydrogénation de composés insaturés.

12. Utilisation du catalyseur décrit dans les revendications 1 à 8, pour l'hydrogénation sélective d'alcynènes en alcadiènes, pour l'hydrogénation sélective d'alcynes, d'alcynènes et d'alcadiènes en alcènes, pour l'hydrogénation sélective de phénylalcynes en phénylalcènes et/ou phénylalcanes et/ou pour l'hydrogénation sélective de phénylalcènes en phénylalcanes.

13. Procédé d'hydrogénation sélective de composés insaturés dans des courants d'hydrocarbure, en phase gazeuse ou liquide, à des températures de l'ordre de 0°C à 180°C et des pressions de l'ordre de 2 à 50 bars, **caractérisé en ce qu'**on effectue l'hydrogénation sélective en une ou plusieurs étapes réactionnelles et **en ce qu'**on utilise dans au moins une étape réactionnelle le catalyseur décrit dans les revendications 1 à 8.

14. Procédé suivant la revendication 13, **caractérisé en ce qu'**on hydrogène de manière sélective de l'acétylène en éthylène dans un courant de C2.

15. Procédé suivant la revendication 13, **caractérisé en ce qu'**on hydrogène de manière sélective du propyne et/ou du propadiène en propylène dans un courant de C3.

16. Procédé suivant la revendication 13, **caractérisé en ce qu'**on hydrogène de manière sélective du 1-butyne, du 2-butyne, du 1,2-butadiène et/ou du vinylacétylène en 1,3-butadiène, 1-butène, cis-2-butène et/ou trans-2-butène dans un courant de C4.

17. Procédé suivant la revendication 13, **caractérisé en ce qu'**on hydrogène de manière sélective du 1-butyne, du 2-butyne, du 1,2-butadiène, du 1,3-butadiène et/ou du vinylacétylène en 1-butène, cis-2-butène et/ou trans-2-butène dans un courant de C4.

18. Procédé suivant la revendication 13, **caractérisé en ce qu'**on hydrogène de manière sélective des composés insaturés et/ou des substituants insaturés de composés aromatiques en composés à saturation plus élevée et/ou en composés aromatiques comportant des substituants à saturation plus élevée dans un courant de C5+.